# EUROPÄISCHE PATENTSCHRIFT

(11) **EP 0 906 280 B1**
(45) Veröffentlichungstag und Bekanntmachung des Hinweises auf die Patenterteilung: **03.05.2000**
(21) Anmeldenummer: 97927060.0
(22) Anmeldetag: 28.05.1997
(51) Int. Cl.: C07D 211/58, C07D 211/72, C07B 63/04

(54) **STERISCH GEHINDERTES 4-AMINO-PIPERIDIN MIT GERINGEM DIMERGEHALT, SEINE HERSTELLUNG UND VERWENDUNG**
STERICALLY HINDERED 4-AMINO-PIPERIDINE WITH A LOW DIMER CONTENT, ITS PRODUCTION AND USE
4-AMINOPIPERIDINE A ENCOMBREMENT STERIQUE AVEC UNE FAIBLE TENEUR EN DIMERE, SA PRODUCTION ET SON UTILISATION

(30) Priorität: 03.06.1996 DE 19622268
(43) Veröffentlichungstag der Anmeldung: 07.04.1999
(73) Patentinhaber: BASF AKTIENGESELLSCHAFT, 67056 Ludwigshafen (DE)
(72) Erfinder: JULIUS, Manfred, D-67117 Limburgerhof (DE); RUST, Harald, D-67435 Neustadt (DE); KRAUSE, Alfred, D-67346 Speyer (DE); SIEGEL, Hardo, D-67346 Speyer (DE); SIEGEL, Wolfgang, D-67117 Limburgerhof (DE); WITZEL, Tom, D-67069 Ludwigshafen (DE)
(86) Internationale Anmeldenummer: EP9702763
(87) Internationale Veröffentlichungsnummer: WO9746528

(56) Entgegenhaltungen:
- EP-A- 0 477 593
- DE-A- 4 239 437
- US-A- 4 316 837

## Beschreibung

Die Erfindung betrifft ein Verfahren zur Verringerung der Dimerenbildung von Piperidinen der Formel I 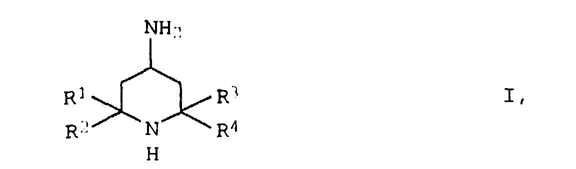 in der R¹ bis R⁴ C₁- bis C₆-Alkyl, R¹ und R² und/oder R³ und R⁴ gemeinsam eine CH₂-Kette mit 2 bis 5 C-Atomen bedeuten. Weiterhin betrifft die Erfindung ein Gemisch, das ein Piperidin der Formel I, 1 bis 1000 ppm eines Dimeren II und 0,001 bis 0,2 Gew.-% eines Reduktionsmittels enthält, und dessen Verwendung zur Herstellung von HALS-Stabilisatoren.

Sterisch gehinderte 4-Amino-piperidine der allgemeinen Formel I werden großtechnisch im allgemeinen aus Aceton oder Acetonderivaten hergestellt. Man kann I einstufig in einer katalytischen Ringschlußreaktion in Gegenwart von Ammoniak und Wasserstoff aus den Verbindungen 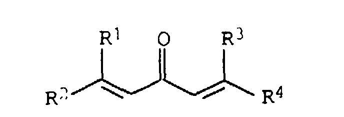 (DE 2 412 750) erhalten oder aus Triacetonaminen III 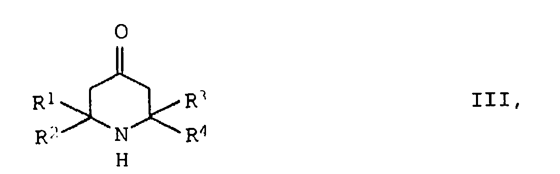 durch aminierende Hydrierung in ein oder zwei Stufen, beispielsweise katalytisch, erhalten (siehe beispielsweise DE 2 040 975, DE 2 349 962, DE 26 21 870, EP 33 529, EP 42 119, EP 303 279, EP 410 970, EP 611 137, EP 623 585 und DE 42 10 311). Die Reinigung des technisch hergestellten sterisch gehinderten 4-Amino-piperidins erfolgt im allgemeinen durch Destillation.

Das sterisch gehinderte 4-Amino-piperidin der allgemeinen Formel I wird vielfältig eingesetzt. Es dient insbesondere als Zwischenprodukt bei der Herstellung von UV-Stabilisatoren für synthetische Polymere. Weiterhin betrifft die Erfindung ein Gemisch, das ein Piperidin der Formel I, 1 bis 1000 ppm eines Dimeren II und 0,001 bis 0,2 Gew.-% eines Reduktionsmittels enthält, und dessen Verwendung zur Herstellung von HALS-Stabilisatoren (HALS = Hindered Amine Light Stabilizer). Es handelt sich dabei meist um Piperidine I, bei denen das Stickstoffatom der 4-Aminogruppe alkyliert oder acyliert ist. (s. R. Gächter, H. Müller (Herausgeber): Taschenbuch der Kunststoffadditive, Carl Hanser Verlag, München, 1979; F. Gugumus, Polym. Degrad. Stabil. 44, 299-322(1994).

Es ist für viele Anwendungen von Wichtigkeit, daß das Piperidin I eine hohe chemische Reinheit besitzt. Dies gilt insbesondere dann, wenn das Piperidin I zur Herstellung von HALS-Stabilisatoren eingesetzt wird, da deren Produktqualität und damit auch die Qualität der stabilisierten Polymeren entscheidend von der chemischen Reinheit abhängt. Insbesondere das Dimere des Piperidins I, das farblos ist, ist ein störendes Nebenprodukt, das sich aus dem Piperidin I schon während der Lagerung bei Raumtemperatur auch unter Schutzgas im Dunkeln in nicht tolerierbaren Mengen bildet.

Verfahren zur Verhinderung der Dimerenbildung des Piperidins sind nicht bekannt.

Aus US 4 316 837 war bekannt, 4-Iminopiperidylgruppen in verzweigten aliphatischen Alkoholen zu den entsprechenden 4-Aminopiperidylhaltigen verzweigten Alkoholen zu reduzieren. Das Dimere II des Piperidins I ist nicht offenbart.

In DD 266 799 wird beschrieben, zur Reinigung Piperidine I in Aceton-/Wasserlösung mit CO₂ umzusetzen, den Niederschlag abzutrennen, mit Aceton zu waschen, anschließend thermisch zu zersetzen und das Produkt durch Destillation zu reinigen. In SU 18 11 527 wird ein anderes Reinigungsverfahren für sterisch gehindertes 4-Amino-piperidin I beschrieben. Das verunreinigte Rohprodukt wird dabei in einem aprotischen Lösungsmittel gelöst, mit Ethylenglykol umgesetzt, das Umsetzungsprodukt destilliert und in mehreren Schritten gereinigt. Beide Verfahren sind außerordentlich aufwendig und kostenintensiv. Sie verhindern in keiner Weise die störende Nachbildung des Dimeren.

Aus EP 477 593 war bekannt, daß man die Eigenfärbung von rohen N-Alkyl-di-alkanolaminen verbessern kann, wenn man ein Metallborhydrid zusetzt und in Gegenwart von Wasser unter bestimmten Bedingungen destilliert. Es handelt sich bei den N-Alkyl-di-alkanolaminen jedoch um eine völlig andere Substanzklasse als die sterisch gehinderten 4-Amino-piperidine der vorliegenden Erfindung. Weiterhin war aus Spec. Chem. 4(2), S. 38-41 (1984) und US 3,159,276, US 3,207,790 und US 3,222,310 bekannt, daß durch Zusatz von Natriumborhydrid zu Ethanolaminen, Ethylenaminen oder aromatischen Aminen die Farbe der Produkte verbessert werden kann. Auch in dieser Schrift wird das Piperidin I, auch ebensowenig ein farbloses Dimeres II erwähnt.

Aufgabe der vorliegenden Erfindung war es daher, die Dimerisierung des Piperidins I zu verhindern bzw. zurückzudrängen.

Demgemäß wurde das oben genannte Verfahren gefunden, das dadurch gekennzeichnet ist, daß man dem Piperidin I 0,001 bis 0,2 Gew.-%, bezogen auf das Piperidin I eines Reduktionsmittels der Formel MXH₄₋ₘYₘ, in der M ein Alkalimetall, NR₄, wobei R gleiche oder verschiedene C₁-C₄-Alkylgruppen bedeuten, oder ein Äquivalent eines Erdalkalimetalls oder ein Äquivalent Zink, X Bor oder Aluminium, Y CN und m 0 oder 1 bedeuten, zusetzt. Weiterhin wurde die folgende Stoffmischung gefunden:

### Piperidin der Formel I

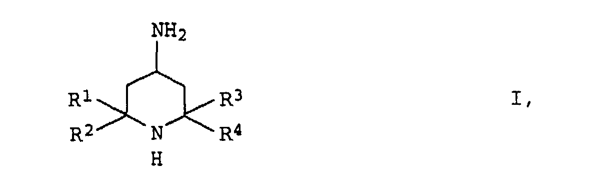

enthaltend 1 bis 1000 ppm einer Verbindung der Formel II, 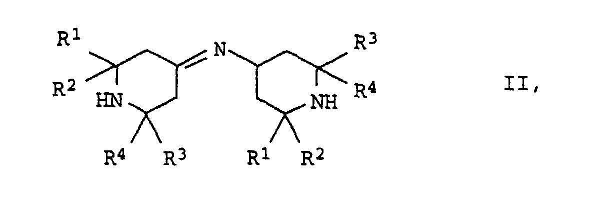 und 0,001 bis 0,2 Gew.-%, jeweils bezogen auf das Piperidin I, eines Reduktionsmittels. Darüber hinaus wurde die Verwendung dieser Stoffmischung zur Herstellung von HALS-Stabilisatoren gefunden.

Weitere Ausprägungen der Erfindung sind den Unteransprüchen zu entnehmen.

Im sterisch gehinderten 4-Amino-piperidin I sind die Reste R¹, R², R³ und R⁴ unabhängig voneinander bevorzugt C₁ bis C₃-Alkyl, insbesondere Ethyl oder Methyl, beispielsweise Methyl.

Als Reduktionsmittel werden Verbindungen MXH_{4;m}Yₘ, in denen M ein Alkalimetall, NR₄, wobei R gleiche oder verschiedene C₁-C₄-Alkylgruppen bedeuten, oder ein Äquivalent eines Erdalkalimetalles oder ein Äquivalent Zink, bevorzugt ein Alkalimetall, insbesondere Natrium oder Kalium, beispielsweise Natrium, und X Aluminium oder insbesondere Bor, Y CN und m 1 oder insbesondere 0 darstellen, eingesetzt. Ein Beispiel ist Natriumborhydrid. In manchen Fällen haben sich auch (R^{a}O)₂TiBH₄ oder (R^{a}O)₃TiBH₄, in denen R^{a} eine C₁-C₄-Alkylgruppe bedeutet, bewährt.

Die Menge des Reduktionsmittels, das dem Piperidin I zugesetzt wird, ist bevorzugt 10 bis 1000 ppm, manchmal bis 500 ppm, insbesondere bis 200 ppm, bezogen auf das Piperidin I. In manchen Fällen kann das Reduktionsmittel auch in Mengen von über 50 ppm, insbesondere über 100 ppm, jeweils bezogen auf das Piperidin I, zugegeben werden.

Seine Zugabeart ist unkritisch. Das Reduktionsmittel kann auf einmal oder chargenweise einer Vorlage, die das Piperidin I enthielt zugegeben und eingemischt werden, beispielsweise durch Rühren. Dieses kann diskontinuierlich oder kontinuierlich geschehen, letzteres beispielsweise auch durch zudosieren des Reduktionsmittels zu einem Produktstrom, der das Piperidin I enthält oder aus ihm besteht. Es ist auch grundsätzlich denkbar, vorgelegte und zugegebene Substanz auszutauschen. Temperatur und Druck sind unkritisch, die Zugabe des Reduktionsmittels kann bei den üblichen Verfahrensschritten der Herstellung des Piperidins I, beispielsweise während oder insbesondere nach dem Destillationsschritt, der der Endreinigung des Piperidins I im Herstellungsprozeß dient, erfolgen. Das Reduktionsmittel kann in Pulverform oder als Lösung zugesetzt werden, beispielsweise gelöst in reinem Piperidin I.

Die Stoffmischung gemäß Anspruch 5 aus Piperidin I, Dimerem II und Reduktionsmittel läßt sich in einfacher Weise herstellen, indem zunächst rohes Piperidin I destillativ vom Dimeren II in gewünschter Weise abgetrennt wird. Dies kann beispielsweise durch Destillation geschehen da das Dimere II einen deutlich höheren Siedepunkt hat als das Piperidin I. Der Fachmann kann leicht, beispielsweise durch gaschromatographischen Analyse den Gehalt an Dimeren bestimmen. Danach wird wie beschrieben das Reduktionsmittel zugesetzt.

Die Herstellung von HALS-Stabilisatoren aus der erfindungsgemäßen Stoffmischung erfolgt im allgemeinen durch Alkylierung oder Acylierung des Piperidins I am Stickstoffatom der 4-Aminogruppe in üblicher Weise.

Mit dem erfindungsgemäßen Verfahren erhält man in einfacher, kostengünstiger Weise reine sterisch gehinderte 4-Aminopiperidine I, die praktisch frei von Dimeren sind. Diese Stabilisierung kann in einfacher Weise bei Raumtemperatur geschehen und führt häufig zu leicht handhabbaren Lösungen, die zumindest mehrere Wochen frei von Dimeren bleiben.

Daraus lassen sich vorteilhafte HALS-Stabilisatoren mit geringem Nebenproduktgehalt herstellen.

### Beispiele

4208 g rohes 4-Amino-2,2,6,6-tetramethyl-piperidin (Triacetondiamin, TAD) mit der Zusammensetzung
85,6 % Triacetondiamin (TAD)
9,0 % H₂O
ca. 0,7 % Tiefsieder
4,7 % Mittel- und Hochsieder
wurden in einer Laborapparatur in einer Kolonne mit 2,4 m Sulzer-CY-Packung (ca. 22 theor. Böden, Nennweite: 43 mm) bei einem Rücklaufverhältnis von 5:1 und einem Druck von 100 bis 40 mbar rektifiziert. Nach der Abtrennung des Wassers im Vorlauf bei einer Temperatur von 43 bis 44°C und einem Druck von 100 mbar wurden im Hauptlauf bei einem Druck von 40 mbar und einer Kopftemperatur von 97 bis 103°C TAD-Reinfraktionen mit einem TAD-Gehalt von > 99,6 % erhalten (Gas-Chromatographie; 30 m Kapillarsäule RTX-5 Amine); Destillationsausbeute: 3022 g (84 %).

Eine TAD Reinfraktion (TAD-Gehalt nach GC: 99,7 %) wurde zum einen ohne Natriumborhydrid-Zusatz und zum anderen nach Zusatz von 1000 ppm an Natriumborhydrid unter identischen Bedingungen gelagert und vermessen. Das Ergebnis zeigt die folgende Tabelle:

| Lagerzeit in Wochen | Zusammensetzung nach GC (Flächen %) | | | |
|---|---|---|---|---|
| | Ohne NaBH₄-Zusatz | | mit NaBH₄-Zusatz | |
| | TAD mer II* | Di- | TAD | Dimer II* |
| 0 | 99,69 | 0,02 | 99,65 | 0,01 |
| 23 | 98,26 | 0,87 | 99,64 | 0,0001 bis 0,001 |

| | | | | |
|---|---|---|---|---|
| * R¹, R², R³, R⁴ = Methyl | | | | |

## Patentansprüche

1. Verfahren zur Verringerung der Dimerisierung von Piperidin der Formel I 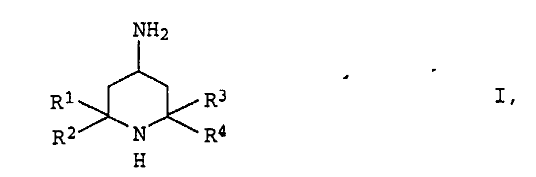 in der R¹ bis R⁴ C₁- bis C₆-Alkyl, R¹ und R² und/oder R³ und R⁴ gemeinsam eine CH₂-Kette mit 2 bis 5 C-Atomen bedeuten, dadurch gekennzeichnet, daß man dem Piperidin 0,001 bis 0,2 Gew.-%, bezogen auf das Piperidin, eines Reduktionsmittels der Formel MXH₄₋ₘYₘ, in der M ein Alkalimetall, NR₄, wobei R gleiche oder verschiedene C₁-C₄-Alkylgruppen bedeuten, oder ein Äquivalent eines Erdalkalimetalls oder ein Äquivalent Zink, X Bor oder Aluminium, Y CN und m 0 oder 1 bedeuten, zusetzt.

2. Verfahren nach Anspruch 1, dadurch gekennzeichnet, daß R¹, R², R³ und R⁴ C₁- bis C₃-Alkyl bedeuten.

3. Verfahren nach Anspruch 2, dadurch gekennzeichnet, daß die Verbindung der Formel MXH₄₋ₘYₘ Natriumborhydrid ist.

4. Gemisch enthaltend
A. Piperidin der Formel I 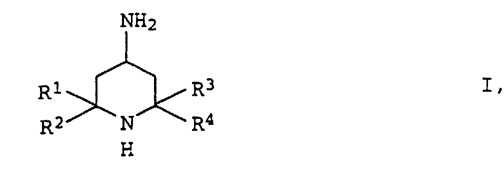 wobei R¹ bis R⁴ C₁- bis C₆-Alkyl, R¹ und R² und/oder R³ und R⁴ gemeinsam eine CH₂-Kette mit 2 bis 5 C-Atomen bedeuten,
B. 1 bis 1000 ppm, bezogen auf A, einer Verbindung der Formel II, 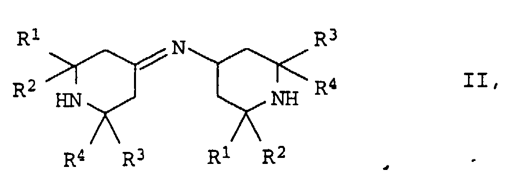 und
C. 0,001 bis 0,2 Gew.-%, bezogen auf A, eines Reduktionsmittels der Formel MXH₄₋ₘYₘ, in der M ein Alkalimetall, NR₄, wobei R gleiche oder verschiedene C₁-C₄-Alkylgruppen bedeuten, oder ein Äquivalent eines Erdalkalimetalls oder ein Äquivalent Zink, X Bor oder Aluminium, Y CN und m 0 oder 1 bedeuten.

5. Gemisch nach Anspruch 4, wobei R¹, R², R³ und R⁴ C₁-bis C₃-Alkyl, bedeuten.

6. Gemisch nach den Ansprüchen 4 oder 5, wobei die Verbindung der Formel MXH₄₋ₘYₘ Natriumborhydrid ist.

7. Verwendung eines Gemisches gemäß einem der Ansprüche 4 bis 6 zur Herstellung von HALS-Stabilisatoren.

## Claims

1. A process for reducing the dimerization of piperidine of the formula I 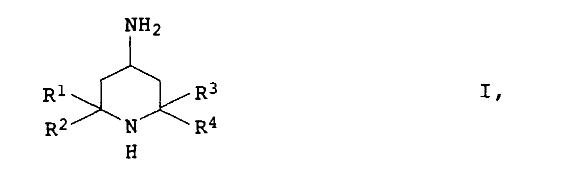 where R¹ to R⁴ are C₁- to C₆-alkyl, R¹ and R² and/or R³ and R⁴ together are a CH₂ chain having 2 to 5 carbon atoms, which comprises adding from 0.001 to 0.2 % by weight, based on the piperidine, of a reducing agent of the formula MXH₄₋ₘYₘ, where M is an alkali metal, NR₄, where R are identical or different C₁-C₄-alkyl groups, or an equivalent of an alkaline earth metal or an equivalent of zinc, X is boron or aluminum, Y is CN, and m is 0 or 1, to the piperidine.

2. A process as claimed in claim 1, wherein R¹, R², R³ and R⁴ are C₁- to C₃-alkyl.

3. A process as claimed in claim 2, wherein the compound of the formula MXH₄₋ₘYₘ is sodium borohydride.

4. A mixture comprising
A. piperidine of the formula I 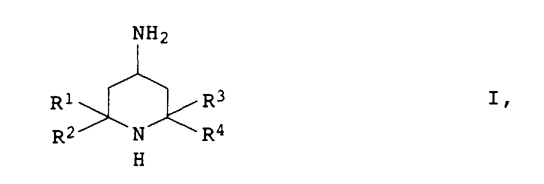 where R¹ to R⁴ are C₁- to C₆-alkyl, R¹ and R² and/or R³ and R⁴ together are a CH₂ chain having 2 to 5 carbon atoms,
B. from 1 to 1000 ppm, based on A, of a compound fo the formula II 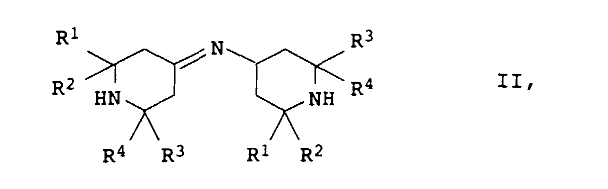 and
C. from 0.001 to 0.2 % by weight, based on A, of a reducing agent of the formula MXH₄₋ₘYₘ, where M is an alkali metal, NR₄, where R are identical or different C₁-C₄-alkyl groups, or an equivalent of an alkaline earth metal or an equivalent of zinc, X is boron or aluminum, Y is CN, and m is 0 or 1,

5. A mixture as claimed in claim 5, where R¹, R², R³ and R⁴ are C₁- to C₃-alkyl.

6. A mixture as claimed in claim 4 or 5, where the compound of the formula MXH₄₋ₘYₘ is sodium borohydride.

7. A method of using a mixture as claimed in any of claims 4 to 6 for the preparation of HALS compounds.

## Revendications

1. Procédé de diminution de la dimérisation de pipéridines de formule I 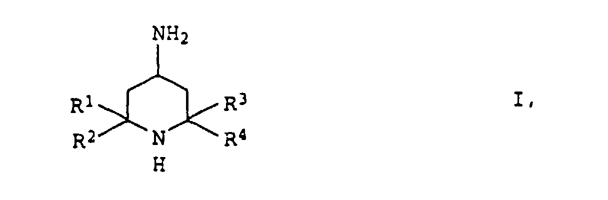 dans laquelle R¹ à R⁴ représentent des groupements alkyle en C₁-C₆, R¹ et R² et/ou R³ et R⁴ représentent ensemble une chaîne CH₂ ayant 2 à 5 atomes de carbone, caractérisé en ce que l'on ajoute à la pipéridine 0,001 à 0,2% en poids, par rapport à la pipéridine, d'un agent réducteur de formule MXH₄₋ₘYₘ, dans laquelle M représente un atome de métal alcalin, NR₄, où R représentent des groupements alkyle en C₁-C₄ identiques ou différents, ou un équivalent d'un atome de métal alcalino-terreux ou un équivalent de zinc, X représente un atome de bore ou d'aluminium, Y représente CN et m vaut 0 ou 1.

2. Procédé selon la revendication 1, caractérisé en ce que R¹, R², R³ et R⁴ représentent des groupements alkyle en C₁-C₃.

3. Procédé selon la revendication 2, caractérisé en ce que le composé de formule MXH₄₋ₘYₘ est le borohydrure de sodium.

4. Mélange contenant
A. des pipéridines de formule I 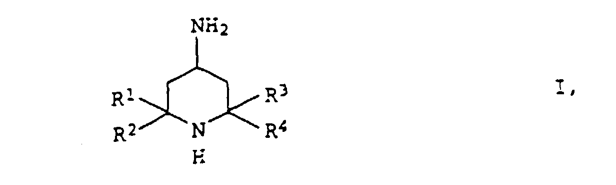 où R¹ à R⁴ représentent des groupements alkyle en C₁-C₆, R¹ et R² et/ou R³ et R⁴ représentent ensemble une chaîne CH₂ ayant 2 à 5 atomes de carbone,
B. 1 à 1000 ppm, par rapport à A, d'un composé de formule II 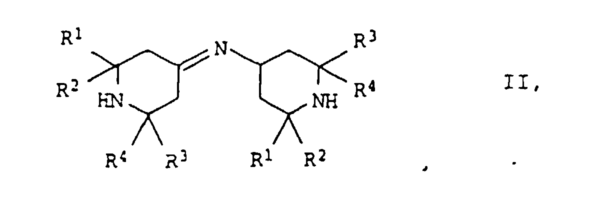 et
C. 0,001 à 0,2% en poids, par rapport à A, d'un agent réducteur de formule MXH₄₋ₘYₘ, dans laquelle M représente un atome de métal alcalin, NR₄, où R représentent des groupements alkyle en C₁-C₄ identiques ou différents, ou un équivalent d'un atome de métal alcalino-terreux ou un équivalent de zinc, X représente un atome de bore ou d'aluminium, Y représente CN et m vaut 0 ou 1.

5. Mélange selon la revendication 4, où R¹, R², R³ et R⁴ représentent des groupements alkyle en C₁-C₃.

6. Mélange selon les revendications 4 ou 5, où le composé de formule MXH₄₋ₘYₘ est le borohydrure de sodium.

7. Utilisation d'un mélange selon l'une quelconque des revendications 4 à 6 pour la préparation de stabilisateurs HALS.
